# EUROPEAN PATENT APPLICATION

(11) **EP 4 344 359 A1**
(43) Date of publication of application: **27.03.2024**
(21) Application number: 22197022.1
(22) Date of filing: 22.09.2022
(51) Int. Cl.: H05G 1/32, A61B 6/00, H05G 1/34, H05G 1/46, H05G 1/60

(54) **IMAGING WITH KVP SWITCHING**

(71) Applicant: Koninklijke Philips N.V., 5656 AG Eindhoven (NL)
(72) Inventor: SCHMÜCKER, Helmut Gerhard, Eindhoven (NL); STRIKER, Timothy, Eindhoven (NL); HYLLA, Arnfinn Richard Christian, 5656AG Eindhoven (NL)
(74) Representative: Philips Intellectual Property & Standards

(57) **Abstract**

The invention concerns a computer-implemented method for controlling operation of an X-ray tube (100) with a high voltage generator (200) assembly for spectral imaging with an imaging system (300). The method comprises receiving (S610) a synchronization signal (SYNC), wherein the synchronization signal (SYNC) relates to a detector integration period. The method further comprises synchronizing (S620), based on the synchronization signal (SYNC), an X-ray tube kVp switching cycle with the detector integration period, and synchronizing (S630), based on the synchronization signal (SYNC), control of an X-ray tube focal spot size and/or focal spot position with the detector integration period. In an embodiment of the invention, the synchronization of the detector integration period with the X-ray tube kVp switching cycle includes triggering control of a tube voltage (HV), and the synchronization of the detector integration period with the X-ray tube focal spot size and/or focal spot position includes triggering control of a focusing current/voltage (FS).

## Description

### FIELD OF THE INVENTION

The invention relates to imaging with an imaging system using kVp switching, and in particular to controlling operation of an X-ray tube with a high voltage generator assembly for spectral imaging.

### BACKGROUND OF THE INVENTION

A computed tomography (CT) scanner generally includes an X-ray tube mounted on a rotatable gantry opposite one or more rows of detectors. The X-ray tube rotates around an examination region located between the X-ray tube and the one or more rows of detectors and emits broadband radiation that traverses the examination region. Electrical power is supplied to the X-ray tube with a high voltage generator.

The one or more rows of detectors detect radiation that traverses the examination region and generate projection data indicative thereof. A reconstructor reconstructs the projection data to generate volumetric image data, which can be displayed, filmed, archived, conveyed to another device, etc.

The detector array includes detector pixels that convert detected x-ray photons into electrical signals indicative thereof. For each revolution of the rotating gantry, the detector pixels detect and convert x-ray photons for a plurality of integration periods, each corresponding to a different angular position range. The time duration of an integration period depends on the rotating gantry rotation speed and the number integration periods for each revolution of the scan. With an integrating detector array, at the beginning of each integration period, the integrators for the detector pixels are reset, and then the integrators receive and integrate the electrical signals over the integration period. The integrated signals form the projection data for that integration period.

The X-ray tube typically includes a cathode with a filament and an anode. A filament current is applied to the filament, which heats the filament, causing the filament to expel electrons (thermionic emission), creating a space charge (or cloud a negative charge) a short distance away from the filament. A peak tube voltage (kVp) is applied across the cathode and the anode and causes a beam of the electrons to accelerate from the cathode and impinge the anode. Electrostatic or magnetic focusing with e.g. grid electrodes or quadrupoles can be applied to control a size of and steer the beam of electrons. An interaction of the electrons with the material of the anode produces heat and radiation, including X-rays, which pass through a tube window, into an examination region, to a detector.

A surface area of the anode that receives the beam of electrons is referred to as a focal spot. The size of the focal spot is one factor that affects the image quality of the volumetric image data. For example, the focal spot size affects the spatial resolution, where a smaller focal spot size results in a greater spatial resolution than a larger focal spot size, e.g., due to less focal spot blur from geometric magnification. The size of the focal spot may depend on the X-ray tube voltage, beam focusing voltage and tube current.

The voxels of the volumetric image data are displayed using gray scale values corresponding to relative radiodensity. The gray scale values reflect the attenuation characteristics of the scanned subject and represent anatomical structures. The detected radiation also includes spectral information as the absorption of a photon by a material of a subject and/or an object is dependent on the energy of the photon traversing the material. Such spectral information provides additional information such as information indicative of the atomic, elemental or material composition of the material. However, the projection data does not reflect the spectral characteristics as the projection data are proportional to the energy fluence integrated over the energy spectrum (e.g., 40 keV to 120 keV), and the volumetric image data will not reflect the energy dependent information.

A CT scanner configured for spectral (multi/dual-energy) imaging leverages the spectral characteristics in the detected radiation to provide further information such as atomic or elemental composition information. In general, a spectral CT scanner is configured to detect different bands of X-ray radiation (instead of just the entire spectrum) and generate projection data for each of the different energy bands (instead of just the entire spectrum). In one instance, this is achieved through so called kVp switching. For example, with a dual-energy configuration, the X-ray tube voltage (i.e. the kVp) may be switched back and forth between two kVp's, such as between a first 80 kVp for odd number data acquisition periods and a second 140 kVp for even number data acquisition periods, or vice versa.

Spectral imaging using fast kVp switching in the high voltage generator may be a promising cost-effective alternative to e.g. spectral imaging with multi-layer energy discriminating scintillator or direct conversion detectors

However, a potential obstacle for extending e.g. existing CT scanners with such a kVp-switching feature, thereby making spectral CT more accessible, is the complexity of required hardware changes. Hence, there is a need for improved simplicity.

### SUMMARY OF THE INVENTION

It is therefore an object of the invention to provide a method and system with reduced cost and/or complexity for imaging using kVp switching.

The invention is defined by the independent claims. Advantageous embodiments are defined in the dependent claims.

According to a first aspect of the invention, there is provided a computer-implemented method for controlling operation of an X-ray tube with a high voltage generator assembly for spectral imaging with an imaging system. The method comprises:
receiving a synchronization signal, wherein the synchronization signal relates to a detector integration period;
synchronizing, based on the synchronization signal, an X-ray tube kVp switching cycle with the detector integration period (e.g. by processing the synchronization signal to generate an output tube voltage for the X-ray tube such that an X-ray tube kVp switching cycle is synchronized with the detector integration period); and
synchronizing, based on the synchronization signal, control of an X-ray tube focal spot size and/or focal spot position with the detector integration period (e.g. by processing the synchronization signal to generate a focusing current/voltage such that control of the X-ray tube focal spot size and/or position with the X-ray tube electron beam optics is synchronized with the detector integration period).

The proposed method may advantageously reduce complexity of spectral imaging with kVp switching in the high voltage generator by processing a common synchronization signal to synchronize both the kVp switching cycle as well as control of focal spot characteristics with information about detector integrations. Data communication between the high voltage generator assembly and other parts of the imaging system may therefore be simplified. This can be particularly advantageous for systems where the high voltage generator assembly is already configured to synchronize the X-ray tube focal spot with the detector integration periods, e.g. during an imaging scan. By processing the same signal for synchronization of kVp switching cycles a spectral imaging function may be achieved with minor adaptations. An X-ray tube kVp switching cycle includes switching and the transitions between at least two kVp's, such as from 80 kV to 140 kV and back to 80 kV to complete one cycle. I.e. a cycle includes at least four phases such as a low plateau kVp, a transition from low to high, a high plateau kVp and a transition from high back to low.

The quality of spectral imaging data reconstruction depends on the spectral separation of applied x-ray in the integration periods with different kVp's as well as the reproducibility of the spectral x-ray distribution in the integration periods with the same kVp. For both conditions, a good synchronization of the kVp switching cycles with the detector integration is important. At the same time, the focal spot control must be well synchronized with the integration periods, on the one hand to keep the focal spot size constant when changing the kVp and on the other hand a good synchronization is a prerequisite for a clear assignment of the different focal spot positions to the integration periods that finally determines spatial resolution.

In an embodiment of the invention, the synchronization of the detector integration period with the X-ray tube kVp switching cycle comprises controlling of a tube voltage, and the synchronization of the detector integration period with the X-ray tube focal spot size and/or focal spot position comprises controlling of a focusing current/voltage (FS). A synchronization signal, such as a synchronization pulse, may advantageously trigger control or adaptation of a tube voltage from the high voltage generator to e.g. initiate or maintain or end a phase of a kVp switching cycle. Similarly, by triggering control of a focusing current/voltage, such as a focusing current/voltage for electron beam optics in the X-ray tube, the focal spot size and/or focal spot position can be adapted or maintained in sync with the detector integration period.

In an embodiment of the invention, the method further comprises:
receiving a predefined voltage switching sequence prior to a spectral imaging scan, wherein the predefined voltage switching sequence is configured to define the length of high voltage phases in multiples of detector integration periods; and
controlling a shape of the X-ray tube kVp switching cycle during the spectral imaging scan, wherein the shape of the X-ray tube kVp switching cycle is based on the predefined voltage switching sequence.

Voltage switching during the spectral imaging scan typically consists of a fast repetition of a tube voltage switch sequence. The detector integrations are synchronized with this sequence. By having a predefined sequence known to the high voltage generator assembly, data communication during the scan can be further simplified. The common synchronization signal may be used to e.g. trigger the start of each sequence, thereby avoiding a need to continuously encode and communicate each stage of the switching cycle.

In an embodiment of the invention, the method further comprises:
receiving a predefined sequence of focal spot sizes and/or focal spot positions prior to a spectral imaging scan, wherein the predefined sequence of focal spot sizes and/or focal spot positions is configured to define the X-ray tube focal spot size and/or focal spot position over multiples of detector integration periods; and
controlling the X-ray tube focal spot size and/or position during the spectral imaging scan, wherein the X-ray tube focal spot size and/or position is based on the predefined sequence of focal spot sizes and/or positions.

Similarly to the previous embodiment, by predefining the sequence of focal spot characteristics at the level of the high voltage generator assembly, data communication with other parts of the system can be simplified during the spectral imaging scan.

In an embodiment of the invention, the method further comprises synchronizing, based on the synchronization signal, an X-ray tube emission current measurement with the detector integration period.

The emission current of the X-ray tube may change when the tube voltage changes and be superimposed by loading currents of the damping capacitance during tube voltage transitions. The accuracy of emission current measurement may thereby be negatively affected during voltage transitions. By synchronizing emission current measurements with detector integration periods and by using the common synchronization signal also with voltage transitions and focal spot characteristics, the accuracy of such measurements can be improved.

In an embodiment of the invention the synchronization signal depends on an angular position of a rotating imaging system gantry.

This is advantageous for spectral image formation since the detector integration, kVp switching and focal spot characteristics are synchronized with the position of the X-ray tube and detector in relation to the patient.

In an embodiment the invention, the synchronization signal is communicated between the imaging system and the high voltage generator assembly through a single digital signal line.

By utilizing a single digital signal line between the system and high voltage generator assembly, the complexity and cost may be reduced.

In an embodiment of the invention, the method further comprises introducing a delay between the synchronization signal and a change in an X-ray tube voltage HV and/or a change in a focusing current/voltage FS. In certain circumstances it may be advantageous to introduce a constant or dynamic delay between a synchronization pulse and the kVp switching and/or change of the focal spot characteristics, in order to optimize imaging. E.g. to adjust the focal spot for kVp ramp up and ramp down phases during a switching cycle.

According to another aspect of the invention, there is provided a computer program element, which, when being executed by a processing unit, is adapted to cause the processing unit to perform the method according to any of the methods as described above.

According to another aspect of the invention, there is provided a computer readable medium having stored thereon the computer program element mentioned above.

According to another aspect of the invention, there is provided a high voltage generator assembly for controlling operation of an X-ray tube for spectral imaging, wherein the high voltage generator assembly is adapted to be connected to the X-ray tube, and the high voltage generator assembly comprises a processing unit configured to carry out any of the methods as described above.

According to another aspect of the invention, there is provided an imaging system for spectral imaging, the imaging system comprising the high voltage generator assembly as mentioned above.

In an embodiment of the invention, the imaging system is a computed tomography system.

These and other aspects of the invention will be apparent from and elucidated with reference to the embodiments described hereinafter.

### BRIEF DESCRIPTION OF THE DRAWINGS

Fig. 1 illustrates a schematic of an X-ray tube for spectral imaging.
Fig. 2 illustrates a schematic of a high voltage generator assembly for controlling operation of an X-ray tube for spectral imaging, according to an embodiment of the invention.
Fig. 3 illustrates a schematic of an imaging system, according to an embodiment of the invention.
Fig. 4 illustrates a sequence diagram according to an embodiment of the invention.
Fig. 5 illustrates a timing diagram according to an embodiment of the invention.
Fig. 6 shows a flowchart representing a method for controlling operation of an X-ray tube, according to an embodiment of the invention.
Fig. 7 shows a flowchart representing a method for controlling operation of an X-ray tube with a predefined voltage switching sequence, according to an embodiment of the invention.
Fig. 8 shows a flowchart representing a method for controlling operation of an X-ray tube with a predefined focal spot characteristic, according to an embodiment of the invention.

### DETAILED DESCRIPTION OF EMBODIMENTS

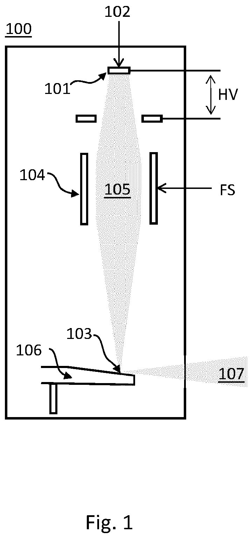

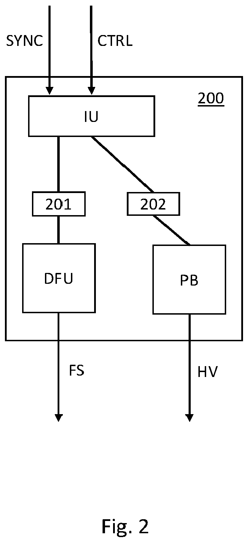

An X-ray tube for spectral imaging is illustrated in Fig. 1. The X-ray tube 100 includes a cathode with a filament 101. A filament current or heating current 102 is applied to the filament 101, causing the filament 101 to expel electrons through thermionic emission. The heating current 102 may be controlled with a high voltage generator 200 as shown in Fig. 2. A tube voltage HV from the high voltage generator 200 is applied in order to accelerate emitted electrons from the cathode filament 101 such that the electrons impinge a rotating anode 106 at a focal spot 103 and generate X-ray radiation 107. The X-ray tube 100 further includes electron beam optics 104, which regulates the size and position of the electron beam 105 between the filament 101 and the anode 106. Thus, the electron beam optics regulates the focal spot 103 size and/or shape and/or position. Also the electron beam optics 103 is controlled by the high voltage generator 200 via a focusing current or voltage FS. Hence the tube voltage HV and the focusing current/voltage FS are controlled by the high voltage generator 200 for X-ray generation with the X-ray tube 100. The electron beam optics 103 in Fig. 1 may be e.g. quadrupoles or other means for magnetic electron beam focusing and positioning and/or grid electrodes for electrostatic focusing and positioning.

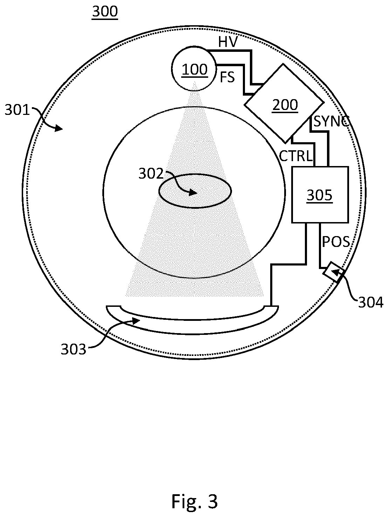

Fig. 2 schematically illustrates a high voltage generator 200, according to an embodiment of the invention. The high voltage generator 200 comprises an interface unit IU, capable of processing data. The interface unit IU may comprise a processing unit. Via a control interface CTRL to the interface unit IU, the high voltage generator 200 may receive and/or communicate control parameters regarding aspects such as, but not limited to, tube peak voltage (kVp), kVp shape, emission current, focal spot size, position etc. The control interface CTRL may be directly interfacing with an imaging system 300, such as illustrated in Fig. 3, or with other user interfaces, such as via a local or remote computer interface etc. to program the high voltage generator 200. The high voltage generator 200 is also configured to receive a synchronization signal SYNC from an imaging system 300, where the synchronization signal is timed to detector integration periods. The synchronization signal SYNC may comprise e.g. synchronization pulses.

The high voltage generator 200 processes the synchronization signal SYNC to generate an output tube voltage HV, with a power block PB, for the X-ray tube 100 such that an X-ray tube kVp switching cycle is synchronized with the detector integration period. The power block PB may also control the tube current and filament current 102. The high voltage generator 200 also processes the synchronization signal SYNC to generate a focusing current/voltage FS such that control of the X-ray tube focal spot size and/or position with the X-ray tube electron beam optics 104 is synchronized with the detector integration period. In Fig. 2, the signal for the electron beam optics 104 is generated with a deflection unit DFU that may drive electromagnetic electron beam optics 104 in the X-ray tube with a focusing current FS. The HV generator 200 might individually add delays 201, 202 to the adjustments of tube voltage HV and/or focusing current FS, e.g. in order to minimize focal spot size deviations during kVp transitions. Thus based on the single synchronization signal SYNC, at least the kVp switching cycle and control of the X-ray tube focal spot size and/or position is synchronized with the detector integration period.

Fig. 3 illustrates an imaging system 300, such as a Computed Tomography (CT) system, according to an embodiment of the invention. The system includes an X-ray tube 100 mounted on a rotatable gantry 301. The X-ray tube 100 is located opposite to a detector 303. The X-ray tube 100 can rotate around an examination region with an imaging subject 302. X-rays from the X-ray tube 100 traverses the examination region and are detected with the detector 303. The imaging system 300 further includes a high voltage generator 200, an imaging system controller 305 and an encoder 304.

The encoder 304 determines the angular position of the X-ray tube 100 and detector 303 and provides an angular position signal POS to the imaging system controller 305. The imaging system controller 305 is connected to the high voltage generator 200 and can communicate with the high voltage generator via a control interface CTRL. The imaging system controller 305 generates a synchronization signal SYNC that relates to a detector integration period, i.e. a period of time when the detector 303 detects radiation, e.g. while rotating through a predetermined angular increment during a scan. The imaging system controller 305 may take the angular position of the X-ray tube 100 and the detector 303 into account when generating the synchronization signal SYNC, since the angular position signal POS is communicated between the encoder 304 and the imaging system controller 305. Based on the synchronization signal SYNC, the voltage generator 200 generates a tube voltage HV and focusing current/voltage FS such that at least the kVp switching cycle and control of the X-ray tube focal spot size and/or position is synchronized with the detector integration period.

In an embodiment of the invention, the kVp switching sequence is pre-defined. In the preparation of a kVp switching imaging scan, the imaging system controller 305 sends a predefined kVp switching sequence to the high voltage generator 200 via the control interface CTRL. Alternatively, the switching sequence is communicated to the high voltage generator 200 via another interface, internal or external to the system 300. The predefined sequence defines the length high voltage phases in multiples of detector integration periods. During the scan, the imaging system controller 305 synchronizes the tube voltage switching with the detector integration periods via the synchronization signal SYNC. E.g. by sending synchronization pulses to the high voltage generator 200 via a single digital signal line. Triggered by the synchronization pulses the high voltage generator 200 starts ramping up and down, possibly with a delay 202, the tube voltage HV in a way that reproduces the predefined kVp switching sequence.

Alternatively or in combination, a sequence of focal spot sizes and/or focal spot positions may be pre-defined in multiples of detector integration periods. In the preparation of the kVp switching imaging scan, the imaging system controller 305 sends a predefined focal spot sequence to the high voltage generator 200 During the scan, the imaging system controller 305 synchronizes the focal spot control with the detector integration periods via the synchronization signal SYNC. E.g. by sending synchronization pulses to the high voltage generator 200 via a single digital signal line. Triggered by the synchronization pulses the high voltage generator starts controlling the size and/or position of the focal spot with the focusing current/voltage FS such that the control of the focal spot reproduces the predefined focal spot sequence.

Emission current of an X-ray tube 100 needs to be monitored in order to ensure patient safety, for example with respect to the X-ray dose, for avoiding tube overloading, etc. In kVp-switching spectral CT applications the tube peak voltage is ramped up and down repeatedly at a high rate. The detector integrations may be limited to phases of the scan in which the tube voltage HV is stable or to phases in which the voltage changes reproducibly during the tube voltage transitions. The emission current also changes when the tube voltage changes and is superimposed by loading currents of the damping capacitance during the tube voltage transitions. The accuracy of emission current measurements may therefore be improved by limiting the measurement to those phases of kVp switching imaging scans in which the detector integration is active. Therefore, in an embodiment of the invention, the synchronization signal SYNC, between the imaging system controller 305 and the high voltage generator 200 is also used to limit the emission current measurement to those scan phases with active detector integration and to mask out parts of the tube voltage transitions that have a negative impact on the emission current measurement. The X-ray generator 200 may need to introduce specific delays for one or both of the functions.

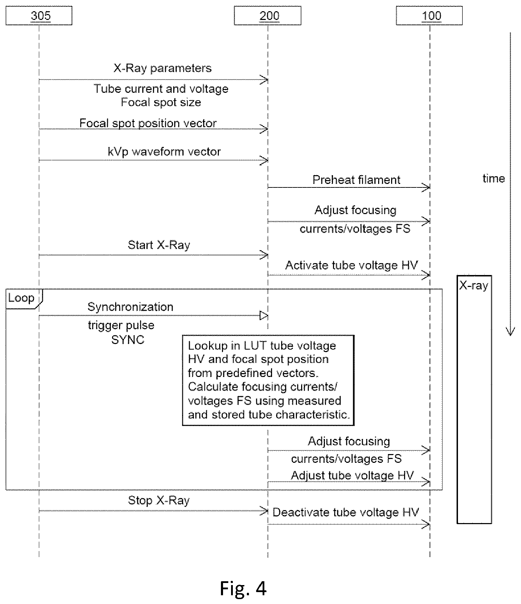

Fig. 4 illustrates a sequence diagram of kVp switching according to an embodiment of the invention. The diagram shows the communication between the imaging system controller 305 and the high voltage generator 200 during an X-ray exposure such as a CT spectral scan.
Prior to the scan, the CT system controller 305 may send exposure parameters to the high voltage generator 200, e.g. via the control interface CTRL: Parameters may include information such as, but not limited to, planned tube voltage and tube current (typical values for low and high kVp are 80 kV and 140 kV); focal spot size; focal spot position vector; the tube voltage (kVp) shape/waveform etc. The waveform e.g. consists of 4 phases: Plateau at low kVp, transition to high kVp, plateau at high kVp and transition back to low kVp. The length of each of the four phases is given in one or several detector integration periods. An integration period depends on the gantry rotation speed. A detector integration period may be on the order of magnitude of 100 µs at full gantry speed. Example times for the four kVp phases are 1000 µs for the plateaus and 300 to 400 µs for the transitions. However transition times may be significantly different depending on changes in the system hardware, such as transition times may be significantly reduced with hardware changes like quadrupole boosting and/or a reduced high voltage damping capacitance.

The high voltage generator 200 adjusts the filament current 102 to start exposure with the selected tube current.

The high voltage generator 200 adjusts the electron optics currents FS for the selected focal spot size at the initial position.

During the exposure, the CT system controller 305 sends synchronization pulses SYNC, which triggers focal spot position adaptation with the focusing current FS and kVp changes with tube voltage HV adaptations, following the predefined sequences.

Electron optic currents FS for given tube voltage HV and current and focal spot position and size may be taken from a look up table (LUT). The LUT may be prepared individually for each X-ray tube or bundle of X-ray tube 100 and high voltage generator 200 during production. The LUT may be prepared or adapted after production and communicated with the high voltage generator 200 via a suitable control interface.

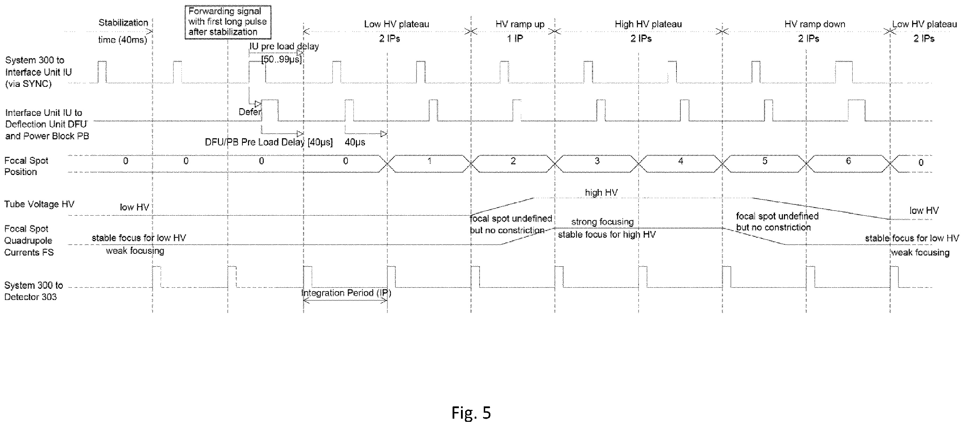

Fig. 5 illustrates a timing diagram according to an embodiment of the invention. The timing diagram shows the synchronization between the imaging system 300 and the interface unit IU of the high voltage generator 200, the step from interface unit UI to deflection unit DFU and power block PB, the resulting focusing current FS, focal spot position and tube voltage HV, as well as detector integration at the system level.

The imaging system controller 305 of the imaging system 300 sends a synchronization trigger pulse SYNC to the interface unit IU ahead in time of the detector integration period, such as e.g. 50 to 100 µs before the beginning of the next integration period, to grant the high voltage generator 200 time to prepare the adjustments.

To allow for resynchronization the system may indicate the start of the change in focal spot position and initiation of the kVp sequence by a long SYNC pulse.

Subsequent steps are triggered by short pulses. The focal spot position vector and the kVp sequence have the same size/length.

The X-ray exposure starts with e.g. a 20 to 40 ms tube current stabilization phase, at the very left of the timing diagram. Processing and forwarding of the SYNC pulses to the deflection unit DFU and the power block PB start with the first long pulse after this stabilization phase.

The high voltage generator 200 might individually delay the adjustments of tube voltage HV and/or focusing currents FS in order to minimize focal spot size deviations during the kVp transitions.

In embodiments of the invention (not shown in Fig. 5), also the emission current measurements are synchronized by the high voltage generator 200, based on the synchronization pulses SYNC.

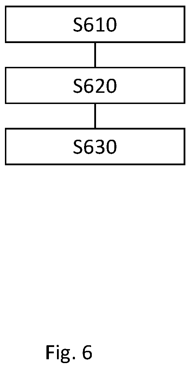

Fig. 6 shows a flowchart representing a method for controlling operation of an X-ray tube, according to an embodiment of the invention. The method comprises:
receiving S610 a synchronization signal SYNC, wherein the synchronization signal relates to a detector integration period;
synchronizing S620, based on the synchronization signal SYNC, an X-ray tube kVp switching cycle with the detector integration period; and
synchronizing S630, based on the synchronization signal SYNC, control of an X-ray tube focal spot size and/or focal spot position with the detector integration period.

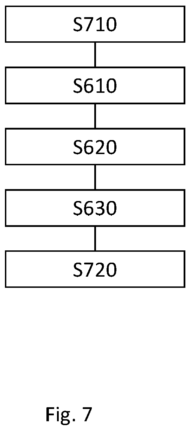

Fig. 7 shows a flowchart representing a method for controlling operation of an X-ray tube with a predefined voltage switching sequence, according to an embodiment of the invention. The method comprises
receiving S710 a predefined voltage switching sequence prior to a spectral imaging scan, wherein the predefined voltage switching sequence is configured to define the length of high voltage phases in multiples of detector integration periods;
receiving S610 a synchronization signal SYNC, wherein the synchronization signal relates to a detector integration period;
synchronizing S620, based on the synchronization signal SYNC, an X-ray tube kVp switching cycle with the detector integration period;
synchronizing S630, based on the synchronization signal SYNC, control of an X-ray tube focal spot size and/or focal spot position with the detector integration period; and
controlling S720 a shape of the X-ray tube kVp switching cycle during the spectral imaging scan, wherein the shape of the X-ray tube kVp switching cycle is based on the predefined voltage switching sequence.

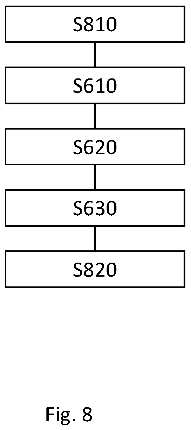

Fig. 8 shows a flowchart representing a method for controlling operation of an X-ray tube with a predefined focal spot characteristic, according to an embodiment of the invention. The method comprises:
receiving S810 a predefined sequence of focal spot sizes and/or focal spot positions prior to a spectral imaging scan, wherein the predefined sequence of focal spot sizes and/or focal spot positions is configured to define the X-ray tube focal spot size and/or focal spot position over multiples of detector integration periods;
receiving S610 a synchronization signal SYNC, wherein the synchronization signal relates to a detector integration period;
synchronizing S620, based on the synchronization signal SYNC, an X-ray tube kVp switching cycle with the detector integration period;
synchronizing S630, based on the synchronization signal SYNC, control of an X-ray tube focal spot size and/or focal spot position with the detector integration period; and
controlling S820 the X-ray tube focal spot size and/or position during the spectral imaging scan, wherein the X-ray tube focal spot size and/or position is based on the predefined sequence of focal spot sizes and/or positions.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

## Claims

1. A computer-implemented method for controlling operation of an X-ray tube (100) with a high voltage generator (200) assembly for spectral imaging with an imaging system (300), the method comprising:
receiving (S610) a synchronization signal (SYNC), wherein the synchronization signal (SYNC) relates to a detector integration period;
synchronizing (S620), based on the synchronization signal (SYNC), an X-ray tube kVp switching cycle with the detector integration period; and
synchronizing (S630), based on the synchronization signal (SYNC), control of an X-ray tube focal spot size and/or focal spot position with the detector integration period.

2. The method of claim 1, wherein
synchronizing (S620) the X-ray tube kVp switching cycle with the detector integration period comprises controlling of a tube voltage (HV), and
synchronizing (S630) control of the X-ray tube focal spot size and/or focal spot position with the detector integration period comprises controlling of a focusing current/voltage (FS).

3. The method of claim 1 or 2, further comprising:
receiving (S710) a predefined voltage switching sequence prior to a spectral imaging scan, wherein the predefined voltage switching sequence is configured to define the length of high voltage phases in multiples of detector integration periods; and
controlling (S720) a shape of the X-ray tube kVp switching cycle during the spectral imaging scan, wherein the shape of the X-ray tube kVp switching cycle is based on the predefined voltage switching sequence.

4. The method according to any of the preceding claims, further comprising:
receiving (S810) a predefined sequence of focal spot sizes and/or focal spot positions prior to a spectral imaging scan, wherein the predefined sequence of focal spot sizes and/or focal spot positions is configured to define the X-ray tube focal spot size and/or focal spot position over multiples of detector integration periods; and
controlling (S820) the X-ray tube focal spot size and/or position during the spectral imaging scan, wherein the X-ray tube focal spot size and/or position is based on the predefined sequence of focal spot sizes and/or positions.

5. The method according to any of the preceding claims, further comprising:
synchronizing, based on the synchronization signal (SYNC), an X-ray tube emission current measurement with the detector integration period.

6. The method according to any of the preceding claims, wherein the synchronization signal (SYNC) depends on an angular position of a rotating imaging system gantry (301).

7. The method according to any of the preceding claims, wherein the synchronization signal (SYNC) is communicated between the imaging system (300) and the high voltage generator (200) assembly through a single digital signal line.

8. The method according to any of the preceding claims, further comprising:
introducing a delay (201, 202) between the synchronization signal (SYNC) and a change in an X-ray tube voltage (HV) and/or a change in a focusing current/voltage (FS).

9. A computer program element, which, when being executed by a processing unit, is adapted to cause the processing unit to perform the method according to any of the preceding claims.

10. A computer readable medium having stored thereon the computer program element of claim 9.

11. A high voltage generator assembly (200) for controlling operation of an X-ray tube (100) for spectral imaging, wherein
the high voltage generator assembly (200) comprises a processing unit configured to carry out a method as claimed in any of claims 1-8.

12. An imaging system (300) for spectral imaging, the imaging system (300) comprising
the high voltage generator assembly (200) as claimed in claim 11, and
the X-ray tube (100).

13. The imaging system (300) according to claim 12, wherein the imaging system (300) is a computed tomography system.
